# EUROPEAN PATENT SPECIFICATION

(11) **EP 3 668 606 B1**
(45) Date of publication and mention of the grant of the patent: **13.09.2023**
(21) Application number: 18739865.6
(22) Date of filing: 17.07.2018
(51) Int. Cl.: A61Q 11/02, A61K 8/02, A61K 8/19, A61K 8/41

(54) **METHOD OF CONTROLLING STAINING OF TEETH DUE TO CATIONIC ANTIMICROBIAL AGENT**
VERFAHREN ZUR STEUERUNG DER FÄRBUNG DER ZÄHNE DURCH KATIONISCHES ANTIMIKROBIELLES MITTEL
PROCÉDÉ DE CONTRÔLE DES DENTS DE COLORATION DUE À LE AGENT ANTIMICROBIEN CATIONIQUE

(30) Priority: 17.08.2017 EP 17186556
(43) Date of publication of application: 24.06.2020
(73) Proprietor: Unilever Global IP Limited, Wirral, Merseyside CH62 4ZD (GB); Unilever IP Holdings B.V., 3013 AL Rotterdam (NL)
(72) Inventor: CHANDRASEKARAN, Sembian, Mumbai 400 099 (IN); IYER, Meenakshi, Mumbai 400 099 (IN); TRIVEDI, Neha, Mumbai 400 099 (IN)
(74) Representative: Tansley, Sally Elizabeth
(86) International application number: PCT/EP2018/069384
(87) International publication number: WO 2019/034352

(56) References cited:
- WO-A1-2013/192463
- WO-A1-2014/102032
- KR-A- 20160 050 126
- US-A- 4 370 314

## Description

### Field of the Invention

The present invention relates to a method of controlling or minimising stains on teeth due to use of oral care products comprising cationic antimicrobial agent.

### Background of the Invention

Oral care compositions usually contain one or more antimicrobial agent, examples include compounds of zinc, Triclosan and Nitrogen-based cationic compounds like Cetyl pyridinium chloride (CPC).

In particular, cationic antimicrobial agents are well known for their action against bacteria in the oral cavity to help prevent, or at least minimize the formation of plaque.

Benzethonium chloride, chlorhexidine and CPC are used more commonly, in mouthwash compositions.

However, their use, in particular, their prolonged use lead to staining or discolouration of teeth. It is believed that the enamel contains high proportion (about 95%) of hydroxyapatite which is a form of calcium phosphate and the phosphate from saliva tends to deposit on teeth which leads to the formation of stains.

This tendency to stain the teeth is responsible, at least in part, for the lack of popularity of oral care compositions which contain such agents.

One way to reduce staining is to use of bleach or oxidants such as peroxide. A bleach acts by oxidizing color bodies and the existing stains. However, this would mean targeting the effect rather than the cause.

WO13192463 A1 (P&G) discloses the use of anti-stain agents from each of at least two of the following chemical groups, viz, anionic agents, carbonyl compounds and nonionic ethoxylated surfactants. The agents are able to reduce aggregation of salivary proteins, either by direct hydrophobic or charged interaction of the anionic agent with the salivary proteins or by the formation of a counter ion with the pyridinium ring of CPC. The anionic agent is more tightly bound to the pyridinium ring than the chloride ion, which then reduces the interaction of CPC with the salivary proteins. Aldehydes react with salivary proteins by forming thioacetals reduce the negative charge density on the surface of the protein and thus, the protein interaction with the positively charged pyridinium ring of CPC. The combination enables stronger interaction between the aldehyde and the protein compared to the interaction between the protein and CPC, as CPC is now tightly bound to a large anionic counter ion and thus less available to interact with the protein. Ethoxylates having the right balance of alkyl and ethoxy units solubilize the salivary proteins, thereby reducing staining.

JP2011201861A (Sunstar INC) discloses the use of one or more kinds of OH-containing compounds selected from the group consisting of thymol, isopropylmethylphenol, terpineol, eugenol, linalool, geraniol and citronellol one or more kinds of diols selected from hexane diol and octane diol to solve the problem of staining.

In JP2011153138A (Sunstar) the problem is solved by including glycyrrhizic acid or its salt.

In JP2011148706A (Sunstar INC) the problem is solved by use of butanediol, pentanediol, hexanediol or octanediol.

In EP0372603 A2 (Colgate Palmolive, 1990), the problem is solved by use of aminoguanidine free base or its water soluble salt.

WO2014102032 A1 (Unilever) discloses a \n oral care composition having: (i) 5 to 60 wt% abrasive; (ii) an anionic surfactant; and, (iii) a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antimicrobial agent attached to the coordinating cation on one of the said external surface plane where the antibacterial agent is a quaternary ammonium material. The disclosed compositions are highly effective at controlling bacterial re-growth, as well as they are equally good at providing immediate control on the bacterial count.

KR20160050126 A (JEJULOVE CO LTD) discloses a toothpaste composition using a scoria powder. Specifically, disclosed is a toothpaste composition for teeth whitening, comprising a scoria powder having a teeth whitening effect as a polishing agent, thereby having a teeth whitening effect.

US4370314 A (Abdul Gaffar, 1983) discloses oral hygiene composition containing a cationic quaternary ammonium antibacterial antiplaque agent and an additive which reduces staining of dental surfaces without unduly diminishing the antibacterial and antiplaque activity of the agent, which additive is an alkali metal hexametaphosphate or mixture thereof with alkali metal bicarbonate. Quaternary ammonium salts include benzethonium chloride and cetyl pyridinium chloride.

However, there is need for further improvised oral care compositions which can solve the problem in an effective yet simple manner and without adversely affecting the antimicrobial action of the cationic agent.

### Summary of the Invention

We have determined that the problem can be solved if the antimicrobial quaternary ammonium compound is in the form of a bipolar composite material comprising a specific type of clay and the ammonium compound.

In accordance with a first aspect is disclosed a bipolar composite material comprising:
(i) a clay whose precursor is an asymmetric 1:1 or 2:1:1 clay particle, comprising alternating tetrahedral and octahedral sheets terminating with a tetrahedral sheet at one external surface plane and an octahedral sheet at another; and,
(ii) antimicrobial quaternary ammonium compound attached to a coordinating cation on one of said external surface planes, for use in an oral care composition, to control staining of teeth due to said ammonium compound.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the term/expression wt% or % by weight, shall mean percentage by weight of the composition, except where indicated otherwise.

### Detailed Description of the Invention

Cationic antimicrobial agents which are known for their propensity to stain teeth include quaternary ammonium salts, chlorohexidine and bis-biquanide salts. While on the one hand, their efficacy against undesired oral microflora makes them suitable for use in oral care compositions such as mouthwash and toothpastes, the side effect is responsible, at least in part, for their lack of popularity. If one still desires to use such products to benefit from their primary effect, the secondary effects needs to be toned down by the use of one or more additional agents disclosed in the compositions forming state of the art. These additional agents may pose some secondary problems of their own such as the possibility of altering the taste, flavor impact or stability of the compositions.

The present invention is a non-obvious solution to the problem, whilst still retaining the essential functional and consumer-relevant attributes of oral care compositions. The problem has been solved by identifying the use of antimicrobial material. Compared to the solutions disclosed in prior art, the solution in the present invention lies in the inclusion of the composite material which acts as a carrier of the cationic antimicrobial agent. The composite material is based on an inert material which does not affect the important properties of the compositions. In WO14102032A1, the Applicant has disclosed that the bipolar antimicrobial material is highly effective at controlling re-growth of bacteria, as well as at providing immediate control on the bacterial count.

It is preferred that median particle size (D50) of this material is 0.1 to 10 µm, more preferably 0.4 to 1 µm and most preferably 0.5 to 0.8 µm. Without being bound by theory, it is believed that this makes more effective use of the antimicrobial agent, thereby providing opportunity for substantial reduction in dosage of the antibacterial agent. While conventional antibacterial agents need an optimised dosage of 0.2 to 0.5 wt%, the antibacterial agent, delivered in the form of the bipolar antibacterial material, is effective even at an (actual) dosage of 0.02 wt% of the oral care compostion, insofar as the antimicrobial effect is concerned.

Lower particle size also provides an effective mechanism for increased the loading of the antibacterial agent, if so desired. Particle size distribution (D50) is also known as the median diameter or the medium value of the particle size distribution, it is the value of the particle diameter at 50 percent in the cumulative distribution. For example, if D50 is 5.8 µm, then 50 % of the particles in the sample are larger than 5.8 µm and 50 percent smaller than 5.8 µm. D50 is usually used to represent the particle size of group of particles. The D50 is the size in microns that splits the distribution with half above and half below this diameter.

It is preferred that in the bipolar composite material comprising a clay, the precursor of the clay is an asymmetric 1:1 clay particle. Preferred 1:1 clays include kaolinite and serpentine subgroups of minerals. The species included within the kaolinite subgroup include but are not limited to kaolinite, dickite, halloysite and nacrite. The species within the serpentine subgroup include but are not limited to chrysolite, lizardite, and amesite. Alternatively, it is also preferred that the precursor of the clay is an asymmetric 2:1:1 clay particle. Preferred 2:1:1 clays include chlorite group of minerals. The chlorite comprises tetrahedral-octahedral-tetrahedral sheets like 2:1 clays, with an extra weakly bound brucite like layer between tetrahedral layers. The tetrahedral sheet preferably comprises coordinating tetrahedral cations of silicon. The tetrahedral sheet may also include isomorphously substituted coordinating tetrahedral cations which are not silicon. Isomorphously substituted coordinating tetrahedral cations include, but are not limited to, cations of aluminum, iron or boron.

It is preferred that the octahedral sheet has coordinating octahedral cations of aluminum. The octahedral sheet may also comprise isomorphously substituted coordinating octahedral cations which are not aluminium. Isomorphously substituted coordinating octahedral cations include cations of magnesium and iron. The antimicrobial agent is attached to the coordinating cations on the exterior side of one of the external surface planes. Accordingly, the antimicrobial agent is attached to coordinating cations on the exterior side of the tetrahedral sheet. Alternatively, the antimicrobial molecule is attached to the coordinating cations on the exterior side of the octahedral sheet. Coordinating cations on the exterior side of each of the tetrahedral and the octahedral surface sheets are attached to the antimicrobial agent, with the proviso that the antimicrobial agent attached to the coordinating cations on the exterior side of the tetrahedral surface sheet is not identical to the molecule attached to the coordinating cations on the exterior side of the octahedral surface sheet. The antimicrobial agent is preferably attached to the coordinating cations on the external surface of the octahedral surface plane and is not preferably attached to coordination cations of non-exterior tetrahedral or octahedral plane or on the interior side of the surface sheets.

It is preferred that the ratio of the clay: antimicrobial agent is from 1:0.001 to 1:1, more preferably from 1:0.001 and 1:0.1 parts by weight of the bipolar composite material.

It is preferred that in the antimicrobial material, the antibacterial agent is attached to coordinating cations on the external surface of the octahedral surface plane.

It is preferred that oral care compositions of the invention comprise 0.1 to 10 wt% of the bipolar antimicrobial material, more preferably 0.5 to 5 wt% material.

Preferably the quaternary ammonium compound is one or more of cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

It is particularly preferred that the antimicrobial agent cetylpyridinium chloride (CPC). Without being bound by theory, it is believed that the primary activity is linked to the cationic charge of its amine group. Thus, cetylpyridinium chloride is attracted to and binds to negatively-charged protein moieties on the cell membrane or cell wall of the microorganism and to tooth surfaces which are also typically negatively charged. The resulting attachment to microorganisms disrupts the cell wall structure causing leakage of the intracellular fluids, eventually killing the associated microorganism. However, as disclosed earlier and in particular with reference to cetylpyridinium chloride, its tendency to stain the teeth has largely been responsible for the lack of popularity of oral compositions which contain for example and in particular, CPC.

It is particularly preferred that precursor of the clay is a 1:1 clay particle. Further preferably the precursor of the clay is kaolinite. It is particularly preferred that the antimicrobial quaternary ammonium compound is cetyl pyridinium chloride. It is further particularly preferred that when the precursor of the clay is kaolinite, the antimicrobial quaternary ammonium compound is cetyl pyridinium chloride.

The description of preferred features applies mutatis mutandis to the other aspects of the invention.

### Oral care compositions

It is preferred that the oral care composition is a toothpaste. Alternatively, the oral care composition is a mouthwash. Other known forms include toothpowder, chewing gums and lozenges, strips and gels.

It is preferred that when the oral care composition according to the invention is a toothpaste, the toothpaste comprises at least one of calcium-based abrasive or silica-based abrasive.

Toothpastes are also known as dentifrices. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically, the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

An oral care composition according to the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice).

Humectants are generally included in toothpastes for a soft, supple mouth feel. Humectants also reduce the tendency of toothpastes to lose moisture. Preferred toothpaste compositions contain 3.5 to 40 wt% humectants. Further preferred compositions have 10 to 40 wt%, more particularly 10 to 20 wt% humectants. A particularly preferred humectant is sorbitol, generally available as 70% aqueous solution. Other preferred humectants include glycerine, maltitol and xylitol. More preferred toothpastes contain glycerine and sorbitol for a lubricated mouth feel, but their cumulative levels should not exceed the disclosed upper limit. Lower humectant content provides an effective way to reduce the cost of the product.

An oral care composition according to the invention will generally contain further ingredients to enhance performance and/or consumer acceptability such as abrasive cleaning agent, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Examples of abrasives include abrasive amorphous silica particles which have a weight mean particle size (d50) ranging from 3 to 15 microns. Preferred abrasive amorphous silica particles for use in the composition of the invention have a weight mean particle size in the range 3 to 6 microns. Preferably, the abrasive amorphous silica particles employed are precipitated silica. Suitable precipitated silicas for use as abrasive amorphous silica particles in the invention are commercially available and include those marketed by PQ Corporation under the trade names SORBOSIL^{®} AC 43, AC77, AC35 and SORBOSIL^{®} AC 33. Mixtures of any of the above described materials may also be used. The level of abrasive amorphous silica particles (as defined above) generally ranges from 0.05 to 5%, preferably from 0.1 to 3%, more preferably from 0.2 to 0.8%, by total weight abrasive amorphous silica particles (as defined above) based on the total weight of the composition.

Preferably the compositions of the invention comprise calcium-based abrasive. A particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Other preferred calcium containing abrasives include dicalcium phosphate (DCP), calcium pyrophosphate and precipitated calcium carbonate (PCC). When a combination of Calcium containing abrasives is used, it is preferred that FGNC is 35 to 100 %, more preferably 75 to 100 % and especially from 95 to 100 % of the total abrasive. In such cases, the balance, most preferably, is PCC.

Furthermore, the compositions of the invention preferably comprise a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the compositions of the invention preferably comprise a surfactant in an amount of from 0.2 to 10% by weight based on the total weight of the dentifrice. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

### Smectite clay

It is preferred that oral care compositions of the invention include a smectite clay which is in addition to the clay present by way of the bipolar antimicrobial material. Smectites constitute a group in the class of natural aluminosilicate minerals known as phyllosilicates or layered silicates. Preferred smectite clay is selected from montmorillonites (bentonites, hectorites and derivatives thereof); purified aluminium magnesium silicates (various grades are commercially available as VEEGUM^{(R)}from R. T. Vanderbilt Company); purified sodium magnesium silicates (commercially available as LAPONITE^{(R)} in various grades); organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof. Aluminium magnesium silicate clays are particularly preferred. An example is VEEGUM^{(R)} HV. The clay tends to swell when exposed to water. Preferred toothpaste compositions contain 0.2 to 3 wt percent clay. More preferred compositions include 0.5 to 1 wt percent clay.

The smectite clay not only plays a role in sensory profile as is believed, but it also plays a role in thickening the composition, as the reduced content of thickening silica otherwise leads to a product with lower viscosity.

Oral care compositions of the invention further preferably comprise a zinc salt, preferably zinc sulphate or zinc chloride, more preferably zinc sulphate heptahydrate. Preferably the level of zinc salt is from 0.05 to 1.0 wt% of the total composition more preferably from 0.1 to 0.5 wt%.

Compositions of the invention may comprise a preservative, a preferred preservative is sodium benzoate.

Preferably the level of preservative is from 0.1 to 1 wt% of the total composition. It is preferred that pH of the composition at 20 °C is from 4 to 10, more preferably 7 to 9.

The compositions of the invention may also comprise a deposition aid. The term "deposition aid" in the context of this invention generally means a material which further aids deposition of whitening agents from the composition. An example is polystyrene sulphonates. Another example is Gantrez^{®} polymers.

The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants mentioned above for dentifrices. The amount of humectant generally ranges from 5 to 20% by weight based on the total weight of the mouthwash and the amount of surfactant generally ranges from 0.1 to 5% by weight based on the total weight of the mouthwash.

Compositions of the present invention (such as in particular dentifrices or mouthwashes) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

Use of the composition in the context of this invention typically involves application of the composition to the oral cavity, for a recommended time, before being expectorated. The preferred time application time being from 10 to 60 seconds.

Various examples illustrative of the invention are presented as follows and are no way to be considered as limiting the scope of the invention.

### Examples

### Example 1: In-vitro teeth staining assay

Two toothpaste compositions were prepared. The compositions are detailed in Table 1.

**Table 1**

| **Ingredient/wt%** | **Toothpaste Composition** | |
|---|---|---|
| | **Control 1** | **Inventive** |
| calcium carbonate | 40 | 40 |
| sorbitol (70%) | 30 | 30 |
| Veegum^{™} HV | 0.8 | 0.8 |
| Bipolar composite material* | -- | 1.5 |
| Kaolinite | 1.5 | -- |
| seme | 0.5 | 0.5 |
| sodium lauryl sulphate | 2.5 | 2.5 |
| sodium silicate | 1.7 | 1.7 |
| water and minors | to 100 | to 100 |

| | | |
|---|---|---|
| Note*: (i) *The bipolar composite material contained kaolinite and cetyl pyridinuim chloride in an amount which made the final amount of CPC to 0.045 wt% by weight of the total toothpaste composition. D50 of the material was 0.5 µm. The material was made in accordance with Example 1 of US2012/0177712 A1 (Unilever). (ii) As cetyl pyridinium chloride (CPC) is highly unstable and prone to react with sodium lauryl sulphate, no experiments could be carried out in a composition which contained inter alia, CPC but not in the form of the bipolar composite material (iii) Therefore, the experiment was repeated with an additional control composition as referred to in Table 2. | | |

The compositions were subjected to in-vitro assay following the Bovine Tooth Model, (without brushing). The procedure was as follows:
This model utilizes extracted bovine teeth mounted on polyacrylate material. Bovine teeth were first bleached with dilute peroxide followed by washing with water. The bleached teeth were incubated with saliva for four hours and dried. The teeth were then imaged to determine their baseline color values (L*a*b*) using digital photography using the white light imaging system (Fuji 2000 Camera). L* represents lightness on the y axis, a* represents chroma (red-green) on the x axis, and b* represents chroma (yellow-blue) on the z axis. The teeth were then incubated in saliva for 18 hours to generate a mature coating of pellicle thereon. The saliva was then removed and the teeth were treated with a slurry of the concerned toothpaste (containing no antimicrobials) for two minutes.

The toothpaste slurry was removed and the teeth were washed with water for one minute. The teeth specimens were then ready for treatment. The teeth specimen were treated with CPC solution (positive control), water (negative control) or CPC test rinse solution for one minute in the presence of saliva. The teeth were then incubated with saliva for 20 minutes at 35 °C. Each specimen was subsequently treated with freshly made aqueous solution of tea for 15 minutes, followed by another washing and incubation with saliva for 20 minutes. A total of six treatment cycles were carried out. After five cycles, the teeth were dried and the L*a*b* values were measured again using photo imaging. Changes in the individual L*, a*, and b* components (ΔE) were calculated by subtracting the L*a*b* measurements of treated teeth from the L*a*b* measurements of untreated and unstained teeth. The total color change (ΔE) was calculated as the square root of the sum of the square of the ΔE values. A greater value of ΔE value indicates that the difference between untreated and treated teeth is less. In other words, it indicates that more of the stain remains on the teeth. Conversely, lower value of ΔE indicates that less amount of stain remains on the teeth after the treatment and therefore, the concerned composition is more likely to find consumer acceptance as compared to the ones where the ΔE is comparatively more.

All tests were carried out with a replicate of four teeth. The observations are summarised in Table 2.

**Table 2**

| | **Toothpaste Compositions** | | |
|---|---|---|---|
| | (Additional Control) Marketed gel toothpaste (Dentosan^{®}) containing 0.2 wt% Chlorhexidine | Control 1 | Inventive |
| ΔE | 3.4 | 2.2 | 2.1 |

The observation about the additional control composition underlines the fact that oral care compositions containing cationic antimicrobial agents (in this case, toothpastes) can stain the teeth. The observation about the Control 1 composition is not surprising considering the fact that this composition did not contain any cationic antimicrobial agent. Therefore, it is expected that the ΔE value would be lower than the Additional Control composition.

However, the observation with the ΔE value of the inventive composition is that the ΔE value is the same as that of the corresponding Control Composition 1. This was a surprising observation because the expectation was that the presence of cetyl pyridinium chloride (in the form of the antimicrobial material) would have led to an increase in the ΔE value to somewhere close to the value observed in the case of the Additional Control composition. Therefore the technical effect of the antimicrobial material was wholly unexpected and surprising. In view of this, it amounts to new use of the material.

## Claims

1. A bipolar composite material comprising:
(i) a clay whose precursor is an asymmetric 1:1 or 2:1:1 clay particle, comprising alternating tetrahedral and octahedral sheets terminating with a tetrahedral sheet at one external surface plane and an octahedral sheet at another; and,
(ii) antimicrobial quaternary ammonium compound attached to a coordinating cation on one of said external surface planes,
for use in an oral care composition, to control staining of teeth due to said ammonium compound.

2. A bipolar composite material for use according to claim 1 in which the antimicrobial quaternary ammonium compound is attached to coordinating cations on the external surface of the octahedral surface plane.

3. A bipolar composite material for use according to claim 1 or 2 wherein the ratio of clay to said antimicrobial quaternary ammonium compound, in said material, is from 1:0.001 to 1:1 parts by weight.

4. A bipolar composite material for use according to any of claims 1 to 3 which is present in the oral care composition at a level from 0.1 to 10 wt % of the total composition.

5. A bipolar composite material for use according to any of claims 1 to 4 wherein median diameter (D50) of said materials is 0.1 to 10 µm.

6. A bipolar composite material for use according to any of claims 1 to 5 wherein said quaternary ammonium compound is one or more of cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

7. A bipolar composite material for use according to any of claims 1 to 6 wherein when said precursor is an asymmetric 1:1 clay particle, said clay belongs to kaolinite or serpentine subgroup.

8. A bipolar composite material for use according to any of claims 1 to 6 wherein, when said precursor is an asymmetric 2:1:1 clay particle, said clay belongs to chlorite subgroup.

9. A bipolar composite material for use according to any of claims 1 to 8 in which the oral care composition is a toothpaste.

10. A bipolar composite material for use according to any one of claims 1 to 8 in which the oral care composition comprises calcium-based abrasive

11. A bipolar composite material for use according to any one of claims 1 to 8 which the oral care composition is a mouthwash.

## Patentansprüche

1. Bipolares Verbundmaterial, umfassend:
(i) einen Ton, dessen Vorläufer ein asymmetrisches 1:1- oder 2:1:1-Tonpartikel ist, umfassend abwechselnde tetraedrische und octaedrische Schichten, die mit einer tetraedrischen Schicht an einer äußeren Oberflächenebene und einer octedrischen Schicht an einer anderen enden; und
(ii) eine antimikrobielle quaternäre Ammoniumverbindung, die an ein koordinierendes Kation auf einer der äußeren Oberflächenebenen gebunden ist,
zur Verwendung in einer Mundpflegezusammensetzung, um das Verfärben der Zähne aufgrund der Ammoniumverbindung zu kontrollieren.

2. Bipolares Verbundmaterial zur Verwendung gemäß Anspruch 1, in dem die antimikrobielle quaternäre Ammoniumverbindung an koordinierende Kationen der äußeren Oberfläche der octaedrischen Oberflächenebene gebunden ist.

3. Bipolares Verbundmaterial zur Verwendung nach Anspruch 1 oder 2, wobei das Verhältnis des Tons zu der antimikrobiellen quaternären Ammoniumverbindung in diesem Material 1:0,001 bis 1:1 Gewichtsteile beträgt.

4. Bipolares Verbundmaterial zur Verwendung nach einem der Ansprüche 1 bis 3, das in der Mundpflegezusammensetzung in einer Menge von 0,1 bis 10 Gew.-% der gesamten Zusammensetzung vorliegt.

5. Bipolares Verbundmaterial zur Verwendung nach einem der Ansprüche 1 bis 4, wobei der mittlere Durchmesser (D50) der Materialien 0,1 bis 10 µm beträgt.

6. Bipolares Verbundmaterial zur Verwendung nach einem der Ansprüche 1 bis 5, wobei die quaternäre Ammoniumverbindung darstellt ein oder mehrere von Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid, Quaternium, Tetrabutylammoniumbromid, Undecylenamidopropyltrimoniummethosulfat, Methylbenzethoniumchlorid, Cetethyldimoniumbromid, Cetromoniumtosylat, Cocotrimoniumchlorid, Dodecylbenzyltrimoniumchlorid, Laurylisochinoliumbromid, Laurylpyridiniumchlorid, Dequaliniumchlorid oder Domiphenbromid.

7. Bipolares Verbundmaterial zur Verwendung nach einem der Ansprüche 1 bis 6, wobei der Vorläufer ein asymmetrisches 1:1-Tonpartikel ist, wobei der Ton zur Kaolinit- oder Serpentin-Untergruppe gehört.

8. Bipolares Verbundmaterial zur Verwendung nach einem der Ansprüche 1 bis 6, wobei der Vorläufer ein asymmetrisches 2:1:1-Tonpartikel ist, wobei der Ton zur Chlorit-Untergruppe gehört.

9. Bipolares Verbundmaterial zur Verwendung nach einem der Ansprüche 1 bis 8 , wobei die Mundpflegezusammensetzung eine Zahnpasta ist.

10. Bipolares Verbundmaterial zur Verwendung nach einem der Ansprüche 1 bis 8, wobei die Mundpflegezusammensetzung Schleifmittel auf Calcium-Basis umfasst.

11. Bipolares Verbundmaterial zur Verwendung nach einem der Ansprüche 1 bis 8, wobei die Mundpflegezusammensetzung ein Mundwasser ist.

## Revendications

1. Matériau composite bipolaire comprenant :
(i) une argile dont le précurseur est une particule d'argile 1:1 ou 2:1:1 asymétrique, comprenant des feuilles tétraédriques et octaédriques alternées terminant avec une feuille tétraédrique sur un plan de surface externe et une feuille octaédrique sur l'autre ; et,
(ii) un composé d'ammonium quaternaire antimicrobien attaché à un cation de coordination sur un desdits plans de surfaces externes,
pour une utilisation dans une composition pour le soin oral, pour contrôler la coloration des dents due audit composé ammonium.

2. Matériau composite bipolaire pour une utilisation selon la revendication 1 dans lequel le composé d'ammonium quaternaire antimicrobien est attaché à des cations de coordination sur la surface externe du plan de surface octaédrique.

3. Matériau composite bipolaire pour une utilisation selon la revendication 1 ou 2 dans lequel le rapport d'argile audit composé d'ammonium quaternaire antimicrobien, dans ledit matériau, est de 1:0,001 à 1:1 parties en masse.

4. Matériau composite bipolaire pour une utilisation selon l'une quelconque des revendications 1 à 3 qui est présent dans la composition pour le soin oral à une teneur de 0,1 à 10 % en masse de la composition totale.

5. Matériau composite bipolaire pour une utilisation selon l'une quelconque des revendications 1 à 4 dans lequel le diamètre médian (D50) desdits matériaux est de 0,1 à 10 µm.

6. Matériau composite bipolaire pour une utilisation selon l'une quelconque des revendications 1 à 5 dans lequel ledit composé d'ammonium quaternaire est un ou plusieurs de chlorure de cétylpyridinium (CPC), chlorure de cétyltriméthylammonium (CTAC), bromure de cétyltriméthylammonium (CTAB), chlorure de benzalkonium (KBC), chlorure de benzéthonium, cétrimide, quaternium, bromure de tétrabutylammonium, méthosulfate d'undécylènamido propyltrimonium, chlorure de méthylbenzéthonium, bromure de cetéthyl-dimonium, tosylate de cétromonium, chlorure de cocotrimonium, chlorure de dodécylbenzyltrimonium, bromure de laurylisoquinolium, chlorure de laurylpyridinium, chlorure de déqualinium ou bromure de domiphène.

7. Matériau composite bipolaire pour une utilisation selon l'une quelconque des revendications 1 à 6 dans lequel lorsque ledit précurseur est une particule d'argile 1:1 asymétrique, ladite argile appartient au sous-groupe de kaolinite ou serpentine.

8. Matériau composite bipolaire pour une utilisation selon l'une quelconque des revendications 1 à 6 dans lequel, lorsque ledit précurseur est une particule d'argile 2:1:1 asymétrique, ladite argile appartient au sous-groupe de chlorite.

9. Matériau composite bipolaire pour une utilisation selon l'une quelconque des revendications 1 à 8 dans lequel la composition pour le soin oral est un dentifrice.

10. Matériau composite bipolaire pour une utilisation selon l'une quelconque des revendications 1 à 8 dans lequel la composition pour le soin oral comprend un abrasif à base de calcium.

11. Matériau composite bipolaire pour une utilisation selon l'une quelconque des revendications 1 à 8 dans lequel la composition pour le soin oral est une eau de bouche.
